Europäisches Patentamt

**European Patent Office**

Office européen des brevets

(11) Publication number: **0 087 686**
**A2**

(12) **EUROPEAN PATENT APPLICATION**

(21) Application number: **83101522.7**

(22) Date of filing: **17.02.83**

(51) Int. Cl.³: **C 12 P 21/00**
**A 61 K 45/02**

(30) Priority: **01.03.82 US 353442**

(43) Date of publication of application:
**07.09.83 Bulletin 83/36**

(84) Designated Contracting States:
**AT BE CH DE FR IT LI LU NL SE**

(71) Applicant: **F. HOFFMANN-LA ROCHE & CO.
Aktiengesellschaft**

**CH-4002 Basel(CH)**

(72) Inventor: **Stein, Stanley
19 Leo Terrace
Bloomfield, N.J.(US)**

(72) Inventor: **Wolfe, Richard, A.
8 Crescent Road
Hopatcong, N.J.(US)**

(74) Representative: **Lederer, Franz, Dr. et al,
Patentanwälte Dr. Franz Lederer Reiner F. Meyer
Lucile-Grahn-Strasse 22
D-8000 München 80(DE)**

(54) **Homogeneous human immune interferon and process therefor.**

(57) Human immune interferon as homogeneous protein and method for its preparation by a) passing a solution of crude human immune interferon through an affinity column and eluting the adsorbed interferon, b) submitting pooled active fractions from step a) to HPLC on a weak cation exchange column using a gradient of increasing salt concentration, c) passing pooled active fractions from step b) through a silica gel permeation column and eluting the interferon with a buffered solution of low salt concentration.

EP 0 087 686 A2

## Homogeneous Human Immune Interferon and Process therefor

The present invention relates to human immune interferon in the novel form of a stable, homogeneous protein and to a method for its preparation. Such homogeneous human immune interferon is therapeutically useful as an antiviral and anti-proliferative agent and serves, in combination with other forms of interferon such as leukocyte interferon, to synergize the acitivity of such other interferons.

Partial purification of human immune interferon is described by Georgiades and Johnson [Methods in Enzymology 78, 536 (1981)]. The steps employed for purification included treatment with Matrex Blue absorbent, the supernatant is treated with controlled-pore glass to absorb the interferon, the interferon is eluted with NaCl-ethylene glycol and the interferon fractions are pooled and chromatographed on a Ultrogel AcA-54 column with elution of the interferon peak with 1M NaCl/18% ethylene glycol. The bulk of interferon activity is eluted in the molecular weight range of 35,000-70,000. The specific activity of the Ultrogel peak fraction was reported to be $10^{6.30}$. This was indicated to be the purest material derived to date and represents a purity of less than about 10%.

Large scale induction and production of human immune interferons is reported by Johnson et al. [Methods in Enzymology, 78, 158 (1981)]. They indicate that highest yields of interferon are obtained from human peripheral lymphocytes derived from normal donors which are induced with the T-cell mitogen staphylococcal enterotoxin A. This procedure provides a median interferon concentration of 1000 units/ml, or $2.5 \times 10^5$ units per 100 ml of blood. Partially purified immune interferon derived from the

Mez/17.1.83

aforesaid starting material is indicated by the authors to be suitable for antibody production and for the initiation of clinical trials in humans but no details are given.

Non-glycosylated human immune interferon in crude form was obtained by the use of tunicamycin by Mizrahl and O'Malley [Methods in Enzymology, 78, 209 (1981)]. The non-glycosylated immune interferon exhibited biological activity but lost its ability to bind to an immobilized lectin (ConA-Sepharose).

Affinity chromatography of human immune interferon was described by O'Malley [Methods in Enzymology, 78, 540 (1981)]. Based on examination of various patient's immune interferon levels indicates that immune interferon plays a role in host defense responses against viral infections and that immunosuppressed hosts are impaired in their ability to mount such a response. Additionally, results obtained using various inducers and a number of different affinity columns indicated that immune interferon is comprised of a heterogeneous population of interferon components with differing degrees of hydrophobicity. Further characterization could not be done without the availability of purified interferon preparations from which contamination masking substances have been removed.

Nathan et al. [Nature 292, 842 (August 27, 1981)] have reported on the production of human immune interferon by a human-T lymphocyte cell line. The interferon was purified using controlled pore glass beads followed by column chromatography with Ultrogel AcA-44. The specific activity of the peak fraction from this column was $1.2 \times 10^6$ (less than 5% pure) and the apparent molecular weight was about 40,000.

Induction of human leukocytes by Con A and partial purification by adsorption to silicic acid and elution with buffer containing ethylene glycol yielded two anti-virally active components which were separable from each other by

- 3 -    0087686

gel filtration. The components (designated 45K and 22K) had apparent molecular weights of about 45000 and about 22000. The 45K component was species specific, relatively sensitive to acid (pH 2) and poorly neutralized by antibodies to human fibroblast interferon. The authors concluded that in all probability the 45K component predominently contained molecular variants of human immune interferon. See Van Damme et al. [Eur. J. Immunol., 11, 937-942 (1981)].

Most recently, Yip et al. [Science 215, 411 (January 22, 1982)] described the treatment of highly concentrated, partially purified human immune interferon preparations with 0.1% SDS at temperatures between 20° and 25°C. They noted that such treatment did not completely destroy biological activity as was previously observed when higher temperatures were used. They recovered two peaks with biological activity from SDS-PAGE analysis with apparent molecular weights of 20,000 and 25,000 and trace activity was observed in the region corresponding to a molecular weight of about 50,000. The authors speculate that under the denaturing conditions employed in the assay a high molecular weight form of the immune interferon dissociates into the two components observed. (See also Yip et al., Proc. Nat. Acad. Sci. USA 79, 1820 (March 1982).

Finally, Goeddel et al. have presented a paper at the Second Annual International Congress for Interferon Research at San Francisco, Calif., on October 21-23, 1981, which described the cloning of a human immune interferon complementary DNA and its expression in three different host-vector systems. Based on the nucleotide sequence of this cloned gene and assuming the proper positioning of the initiation of the expressed protein sequence, the putative human immune interferon is composed of 146 amino acids with a total molecular weight of approximately 17,000. According to Gray et al. [Nature 295, 503 (February 11, 1982)] the amino acid composition derived from the nucleotide sequence is as follows:

| Ala | 8 | Gln | 10 | Leu | 10 | Ser | 11 |
| Arg | 7 | Glu | 9 | Lys | 20 | Thr | 5 |
| Asn | 10 | Gly | 5 | Met | 4 | Trp | 1 |
| Asp | 10 | His | 2 | Phe | 10 | Tyr | 5 |
| Cys | 2 | Ile | 7 | Pro | 2 | Val | 8 |

Crude human immune interferon useful in the practice of the present invention can be derived from any convenient source. One suitable source of crude human immune interferon is from induced normal leukocytes. Enhanced yields and titers of human immune interferon can be obtained by innoculating a cell culture medium supplemented with serum; such as fetal calf serum and an agent which stimulates cell proliferation and differentiation and allowing the mixture to incubate for a period sufficient for the cells to undergo at least one doubling. A suitable supplemented cell culture medium for this process is RPMI 1640 medium, an article of commerce, containing about 2-10% fetal calf serum. The supplemented medium may also contain conventional added components useful for cell culture such as, for example, a buffer, e.g. 12.5 to 25 mM Hepes (N-2-hydroxyethyl-piperazine-N'-2-ethane sulfonic acid). The cell proliferation stimulating agent useful in the practice of the instant invention is a phytohemagglutinin (PHA) added in an amount in the range of from about 0.1 to 10 μg/ml. The incubation temperature employed should be preferably about 37°C. Generally, the cells will undergo 1-2 doublings within about two days.

Upon completion of the incubation period the cells are induced. Useful inducing agents which can be employed are well known in the art and include specific antigens or non-specific mitogens. Examples of such inducing agents include phytohemagglutinins (PHA), concanavalin A (Con A), pokeweed mitogen (PWM), purified protein derivative (PPD), tetanus toxoid (TT), vaccinia virus antigen (VM), staphylococcal enterotoxin A, the phorbol esters such as 12-O-tetra-decanoylphorbol 13-acetate (TPA) and the like. It has generally been found that induction methods using specific antigens which are usually added to cultures containing lymphocytes previously sensitized to the antigen result in comparatively poor yields of immune interferon. Thus induction with the T cell mitogens is preferred. Particularly preferred among the mitogens are the phytohemagglutinins, most preferred being phytohemagglutinin-L in conjunction with TPA as described by Yip et al., Proc. Natl. Acad. Sci. USA 78, 1601 (1981).

The PHA is used at a concentration in the range of from about 1 to 10 µg/ml while the TPA is used at a concentration in the range of from about 5 to 20 ng/ml. The phorbol ester is added to the medium first followed after a period of from 2 to 3 hours by the PHA. The incubation period may run for from 48 to 120 hours, preferably about 96 hours. They are then pelleted by centrifugation and the immune interferon containing supernatant is filtered to remove any cellular debris, most preferably through a filter of 3 micron pore size. By use of such procedure for cell proliferation and induction it is possible to substantially increase the yield and titer of the human immune interferon which can be produced per unit cell population when compared to procedures heretofore known in the art. Thus, for example, the average of titers of 45 preparations was about 13,000 units/ml.

An alternate source of crude human immune interferon is derived from recombinant microorganisms which have been transformed with an expression vector containing the human immune interferon gene in operative condition by employing recombinant DNA techniques now well known in the art. The recombinant microorganisms can be fermented, harvested and the immune interferon extracted from the cells by conventional procedures such as, for example, in analogy to the procedures described by Goeddel et al., Nature 287, 411 (1980); Wetzel et al, Journal of Interferon Research 1, 381 (1981) and Gray et al., Nature 295, 503 (1982). The resulting cell free extract can then be processed in the same
manner as described hereafter for supernatant medium derived
from induced leukocytes. It should be noted, however, that
immune interferon, unlike leukocyte interferon is labile to
high temperatures and acidic or basic pH and thus care must
be exercised with the preparation of the crude materials to
avoid such extreme conditions.

The process aspect of the present invention involves
a three step procedure for purifying human immune interferon from the cell free supernatant or extract. In the
first step the human immune interferon is selectively
adsorbed onto an affinity column, the unadsorbed impurities are
washed off the column and then the human immune interferon
is eluted in more highly purified form. The affinity column
purification of human immune interferon is already known
in the art. The specific column materials and elution conditions are not narrowly critical to the practice of the
present invention and thus any of the procedures for affinity chromatography used in the art may be employed, e.g.,
use of controlled-pore plass or a supported lectin column.
A preferred affinity chromatography procedure for use in
the three step process of the invention, as far as naturally
occurring interferon is concerned, utilizes a column of
concanavalin-A Sepharose, which is an article of commerce,
which preferably has been pre-washed with phosphate buffered
saline (PBS) or a culture medium, such as a minimal essential medium. After loading the column and washing with at
least one column volume of PBS or said culture medium the
human immune interferon can be eluted with a dilute solu-
tion of buffered α-methyl-D-mannoside, preferably having a
concentration of about 0.2M.

The pooled active fractions from the affinity chromatography step are then subjected to a novel high performance
weak cation-exchange liquid chromatography step. In this
second step a carboxymethyl silica column (CM) is prepared by washing with a comparatively concentrated, e.g. 1 to 2 M salt solution such as, for example, an alkali metal halide, phosphate or sulphate, preferably KCl. The column is then re-equilibrated with an aqueous buffer preferably phosphate buffer, pH 7.5, or with water and loaded with the pooled active fractions of the affinity column step diluted with water. After washing with water or a low salt concentration solution until the protein concentration observed in the effluent returns to background levels, the column is eluted with an increasing salt concentration gradient. Active fractions are detected by bioassay and are pooled. The size of the column, the flow rates employed and the buffers used are not narrowly critical to the successful practice of the invention. It should be noted that the salt solution selected should not interfere with the methodology employed for protein detection. A preferred system utilizes fluorescamine analysis of the protein and compatible salt gradient made from 0 to 1 molar potassium chloride in buffer solution, such as dilute potassium phosphate, pH 7.5.

The active fractions from the carboxymethyl silica column were pooled and concentrated. A preferred method for concentration of the immune interferon containing solution is by utilizing a filter cone, most preferably a CF 25 filter cone which has been prewashed with 0.4 M KCl, 25 mM potassium phosphate, pH 7.5, and $H_2O$. The concentrated solution is then injected into a pre-equilibrated silica gel permeation column and eluted with a dilute buffered salt solution. As before, the salt which may be employed is not narrowly critical and may be selected from any alkali metal halide, phosphate or sulphate, preferably potassium chloride at a concentration in the range of 0.01 to 1.0M. The buffer used in this step is a dilute alkali metal phosphate buffer, most preferably potassium phosphate, pH 7.5. The purified human immune interferon elutes in a single major protein peak with the bioactivity corresponding to this protein peak. Depending on the state of the crude immune interferon used as starting material and the efficiency of the purification steps it may be possible to obtain homogeneous product at this point. However, if the preparation is still not completely purified, it is possible to reconcentrate the active fractions and then repeat the chromatography on either the TSK or CM column to achieve homogeneity.

Fractions from both the carboxymethyl silica and silica gel permeation columns were assayed in a cytopathic effect (CPE) inhibition assay, such as described in U.S. Pat. No. 4,241,174, issued December 23, 1980, using both Hep-2 (human) and MDBK (bovine) as the assay cells. Antiviral activity was observed with fractions from both columns on the human Hep-2 line but not when the same fractions were tested against the bovine MDBK cells. This species specificity is indicative of the presence of human immune interferon and rules out the presence of any appreciable amounts of human leukocyte or fibroblast type interferons which would provide a positive assay against the bovine MDBK cells.

The homogeneous human immune interferon obtained by the practice of the aforesaid process aspect of the invention exhibited a specific activity of about $1 \times 10^8$ units/ mg protein. The value for the specific activity is only approximately due to inherent variability of the assay as well as to the unavailability of a human immune interferon reference standard. The apparent molecular weight of the homogeneous protein is about 45,000 daltons as determined by polyacrylamide gel electrophoresis in sodium dodecyl sulfate. This molecular weight is also in agreement with its migration on the TSK column.

Interferons have exhibited antiviral, antitumor, growth inhibition and immunosuppression activity. These activities have been obtained even at the clinical level using dosages $1-10 \times 10^6$ units daily using relatively crude preparations, less than 1% of which was human interferon. More recently highly purified recombinant human leukocyte interferons A and D (IFLr-A and IFLr-D) have entered clinical trials. Ascending dosage tests with IFLr-A have demonstrated that single doses as high as 198 million units can be tolerated. Generally therapeutic daily dosages in the range of from about 10-100 million units can be employed, most preferably, about 50 million units. In direct analogy to the above homogeneous human immune interferon can be employed in the same dosage range. It is also possible to utilize more than one type of human interferon in concurrent therapy. Suitable dosage regimens to utilize, for example, human immune interferon in conjunction with a human leukocyte interferon such as IFLr-A will suggest themselves to one skilled in the art. Use of such concurrent therapy results in a synergistic enhancement of activity of the interferons and reduces the amount needed to achieve the desired therapeutic effect.

The process and product aspects of this invention are further illustrated by reference to the following Examples:

## Example 1

Blood from 7-10 normal human donors (approx. 50 ml/ donor) is collected by leukophoresis. Each milliliter of leukophoresed blood yields 15 x $10^6$ leukocytes. Leukocytes are separated from red blood cells by sedimentation in 2% hydroxyethyl starch. The leukocytes are aspirated from the upper phase and pelleted by centrifugation at 500 x g.

The leukocytes are then inoculated at $10^6$ cells/ml in 5-8 liters of RPMI 1640 medium (Gibco) supplemented with 2-10% fetal calf serum, 25 mM Hepes and 0.2-0.5 µg/ml of PHA-L. The PHA stimulates cell proliferation. The culture is incubated for two days at 37°C in a 16 liter flask. The cells undergo 1-2 doublings during this incubation.

The culture is induced, after addition of an equal volume of fresh medium with TPA and PHA as described. The phorbol ester TPA is added to the culture vessel at 10 ng/ml for 3 hours followed by PHA at 2 µg/ml. The induction proceeds for about 96 hours. The cells are then pelleted by centrifugation at 1000 x g and the immune interferon containing supernatant is filtered (3 micron) to remove cellular debris. The filtered solution is now ready for purification.

All of the following steps were performed in the cold at a temperature of about 4°C.

The conditioned medium obtained as described above was sequentially filtered again through a 3 micron and then through a 0.22 micron Nalgene filter. It was then applied to a column of concanavalin-A Sepharose (Con A) (Pharmacia Fine Chemicals or Sigma Chemical Co.) which had previously been washed with either Dulbecco's phosphate buffered saline (PBS) (Grand Island Biological Supply Co.) or a F-11 culture medium (Grand Island Biological Supply Co.). Typically 35 ml bed volume of Con-A was used to adsorb the immune interferon from 1 liter of conditioned medium. The column was then washed with at least one column volume of PBS or F-11 medium. The interferon was then eluted with 0.2M α-methyl-D-mannoside in PBS and the active fractions were pooled. The stability of such active fractions is shown below in Table 1.

## Table 1

Stability of Con A-Purified Immune Interferon at 4°C

| Week | Observed Titer (Units/ml) |
|------|---------------------------|
| 0 | $3.0 \times 10^5$ |
| 1 | $3.1 \times 10^5$ |
| 2 | $1.5 \times 10^5$ |
| 3 | $7.7 \times 10^4$ |
| 5 | $4.1 \times 10^4$ |
| 8 | $4.1 \times 10^5$ |
| 11 | $2.0 \times 10^5$ |
| 12 | $6.1 \times 10^5$ |

### Assay

All immune interferon samples were titrated using the CPE reduction assay as described in U.S. 4,241,174 with the exception that Hep-2 cells (ATCC No. CCL 23) were used as the assay cell. Assays were challenged with vesicular stomatitis virus (VSV) after a 6 hour incubation of the interferon sample and the assay cells. All immune interferon titers are adjusted to reference units using the NIH (G-023-901-527) leukocyte interferon standard.

The next purification step utilized a high performance weak cation-exchange liquid chromatography column. CM 200 resin, 10μm (Separation Industries, Orange, N.J.) was packed into a 25 cm x 0.46 cm I.D. column and prepared for use by cleaning with 2M KCl and re-equilibrating with 25 mM phosphate buffer, pH 7.5, or with distilled, deionized water (this quality of water is used throughout this procedure). The active, pooled eluate from the Con-A column was diluted with an equal volume of water and pumped at 1.5 ml/min onto the CM column. The column was then washed with 25 mM potassium phosphate, pH 7.5, until protein in the column effluent was down to background levels, as determined by automatic monitoring of the column with fluorescamine. The column was then eluted at 0.5 ml/min with a gradient of increasing KCl (0 to 1M) in 25 mM potassium phosphate buffer, pH 7.5. The interferon eluted from the column near the middle of the salt gradient and active fractions were determined by bioassay.

The active fractions from the CM column were pooled and concentrated on a prewashed CF 25 filter cone (Amicon Corp.). The concentrated solution (0.5 ml or less) was then injected into a TSK 250 silica gel permeation column (30 x 0.75 cm) (Bio-Rad Laboratories) which was pre-equilibrated and eluted with 0.3 M KCl, 25 mM potassium phosphate, pH 7.5, at 24 ml/hr and fractions were collected at 1 minute intervals. The activity corresponded to a peak of protein. Homogeneous immune interferon may be obtained in one or more fractions at this stage depending on the starting material and the efficiency of the earlier steps. If necessary impure fractions can be reconcentrated and rechromatographed on the TSK or CM column to achieve homogeneity.

Amino acid analysis of homogeneous human interferon prepared from induced human leukocytes as described above was carried out using post column detection with fluorescamine. Samples were dried in vacuo and hydrolyzed in 6N HCl containing 4% thioglycolic acid for 24 hours in vacuo at 110°C. For cysteic acid analysis, samples were treated with performic acid for 2 hours at 0°C, dried and hydrolyzed in 6N HCl for 24 hours in vacuo at 110°C. The amino acid analysis for two samples (one sample in duplicate) is given below in Table 2. It should be noted that the analysis is not corrected for losses of certain sensitive amino acids such as tryptophan, threonine and serine) nor for incomplete hydrolysis.

## Table 2

### Amino acid composition of human immune interferon

| | Run | | | |
|---|---|---|---|---|
| | A | B | C | Average |
| Asp | 49.7 | 32.9 | 32.1 | 38.2 ± 7.6 |
| Thr | 28.4 | 21.2 | 22.5 | 24.0 ± 2.9 |
| Ser | 26.0 | 19.4 | 19.1 | 22.5 ± 3.3 |
| Glu | 51.0 | 37.9 | 41.9 | 43.6 ± 5.1 |
| Pro* | | 18.6 | 24.3 | 21.5 ± 3.4 |
| Gly | 25.2 | 22.9 | 20.9 | 23.0 ± 1.5 |
| Ala | 26.7 | 23.2 | 24.3 | 24.7 ± 1.3 |
| Val | 25.1 | 24.3 | 24.2 | 24.5 ± 1.7 |
| Met | 4.0 | 3.9 | 4.3 | 4.1 ± 0.2 |
| Ile | 18.5 | 18.4 | 17.9 | 18.3 ± 0.3 |
| Leu | 39.4 | 40.0 | 41.2 | 40.2 ± 0.7 |
| Tyr | 10.0 | 10.0 | 10.8 | 10.3 ± 0.4 |
| Phe | 17.8 | 17.8 | 18.5 | 18.0 ± 0.3 |
| His | 5.3 | 5.2 | 6.4 | 5.6 ± 0.5 |
| Lys | 23.8 | 32.3 | 25.0 | 27.0 ± 3.5 |
| Arg | 20.0 | 22.2 | 22.0 | 21.4 ± 0.7 |
| Trp | 1.8 | 2.7 | 2.2 | 2.2 ± 0.3 |
| Cys | 5.9 | | 4.0 | 5.0 ± 1.0 |

* Values may be too high due to artifact

As pointed out above, the specific activity observed for homogeneous human immune interferon is about $1 \times 10^8$ units/mg and the apparent molecular weight of the homogeneous protein is about 45,000 daltons according to polyacrylamide (10-20% gradient) gel electrophoresis in SDS using an approximately 100 ng load and employing silver staining.

Example 2

Parenteral dosage form with homogeneous human immune interferon

Homogeneous human immune interferon for injection is a lyophilized preparation in sterile vials. Each vial contains approximately 15 mcg of homogeneous immune interferon. The lyophilized cake is reconstituted prior to use with 1 ml of sterile water for injection. This preparation is suitable for intramuscular injection and not for intravenous use. The formulation can contain the following ingredients:

| Ingredients | Quantity per Vial | Reasonable Variation per Vial |
|---|---|---|
| Homogeneous human immune interferon | 15 mcg | 7-23 mcg |
| Sodium chloride | 9 mg | 7-11 mg |
| Phenol * | 3 mg | 2-4 mg |
| Human serum albumin | 5 mg | 3-7 mg |
| Water for injection * | 1 ml | 1-2 ml |

* Mostly lost by volatilization during lyophilization.

What we claim is:

1.    Human immune interferon as a homogeneous protein.

2.    The human immune interferon of claim 1 which is purified from naturally occurring material.

3.    The homogeneous human immune interferon of claim 1
or claim 2 which has an apparent molecular weight of about
45,000 daltons on SDS-PAGE and an average amino acid
composition as follows:

| | | | | |
|---|---|---|---|---|
| Asp | $38.2 \pm 7.6$ | Ile | $18.3 \pm 0.3$ |
| Thr | $24.0 \pm 2.9$ | Leu | $40.2 \pm 0.7$ |
| Ser | $22.5 \pm 3.3$ | Tyr | $10.3 \pm 0.4$ |
| Glu | $43.6 \pm 5.1$ | Phe | $18.0 \pm 0.3$ |
| Pro | $21.5 \pm 3.4$ | His | $5.6 \pm 0.5$ |
| Gly | $23.0 \pm 1.5$ | Lys | $27.0 \pm 3.5$ |
| Ala | $24.7 \pm 1.3$ | Arg | $21.4 \pm 0.7$ |
| Val | $24.5 \pm 1.7$ | Trp | $2.2 \pm 0.3$ |
| Met | $4.1 \pm 0.2$ | Cys | $5.0 \pm 1.0$ |

4.    The human immune interferon as claimed in any one of
claims 1 to 3 when used as a pharmaceutically active agent.

5.    The human immune interferon as claimed in any one of
claims 1 to 3 when used as an antiviral, antitumor, growth
inhibiting and/or immunosuppressive agent.

6.    A method for increasing the purity of human immune
interferon which method comprises passing a solution containing partially purified human immune interferon onto a
high performance weak cation exchange liquid chromatography column and eluting active fractions containing
human immune interferon of enhanced purity from the column
by use of a gradient of increasing salt concentration.

7.    The method of claim 6 wherein said weak cation exchange liquid chromatography column is carboxymethyl silica.

8.    The method of claim 6 wherein said column is eluted with a 0-1M gradient of KCl in potassium phosphate buffer, pH 7.5.

9.    A method for increasing the purity of human immune interferon which method comprises passing a solution containing partially purified human immune interferon onto a silica gel permeation column and eluting active fractions containing human immune interferon of enhanced purity from the column by use of a buffered solution containing a low salt concentration.

10.    The method of claim 9 wherein said elution solution is 0.3M KCl in 25mM potassium phosphate buffer, pH 7.5.

11.    A method for preparing homogeneous human immune interferon which method comprises the following process steps in combination:

a)    passing a solution of crude human immune interferon trough an affinity column which adsorbs said interferon, washing non-adsorbed impurities from said column and eluting active fractions containing human immune interferon from said column;

b)    passing pooled active fractions from step a) onto a high performance weak cation exchange liquid chromatography column and eluting active fractions containing human immune interferon of enhanced purity from the column by use of a gradient of increasing salt concentration;

c)    passing pooled active fractions from step b) onto a silica gel permeation column and eluting active fractions containing human interferon of enhanced purity from the column by use of a buffered solution containing a low salt concentration and, if necessary,

0087686

d)   recycling pooled active fractions from step c) through step b) or step c) to provide homogeneous human interferon.

12.   The method of claim 11 wherein said affinity column in process step a) is concanavalin—A Sepharose, the crude human immune interferon is naturally occurring material and the active fractions are eluted with a buffered α-methyl-D-mannoside solution.

13.   The method of claim 11 wherein said weak cation exchange liquid chromatography column of step b) is carboxymethyl silica and said column is eluted with a 0-1M gradient of KCl in potassium phosphate buffer, pH 7.5.

14.   The method of claim 11 wherein said silica gel permeation column is eluted with a solution which is 0.3 M KCL in 25 mM potassium phosphate buffer, pH 7.5.

15.   A parenteral dosage form comprising a therapeutically effective amount of homogeneous human immune interferon and a pharmaceutically acceptable parenteral carrier material.

1 7. 0087686

## Claims for Austria

1.    A method for increasing the purity of human immune interferon which method comprises passing a solution containing partially purified human immune interferon onto a high performance weak cation exchange liquid chromatography column and eluting active fractions containing human immune interferon of enhanced purity from the column by use of a gradient of increasing salt concentration.

2.    The method of claim 1 wherein said weak cation exchange liquid chromatography column is carboxymethyl silica.

3.    The method of claim 1 wherein said column is eluted with a 0-1M gradient of KCl in potassium phosphate buffer, pH 7.5.

4.    A method for increasing the purity of human immune interferon which method comprises passing a solution containing partially purified human immune interferon onto a silica gel permeation column and eluting active fractions containing human immune interferon of enhanced purity from the column by use of a buffered solution containing a low salt concentration.

5.    The method of claim 4 wherein said elution solution is 0.3M KCl in 25mM potassium phosphate buffer, pH 7.5.

6.    A method for preparing homogeneous human immune interferon which method comprises the following process steps in combination:

a)    passing a solution of crude human immune interferon through an affinity column which adsorbs said interferon, washing non-adsorbed impurities from said column and eluting active fractions containing human immune interferon from said column;

b)    passing pooled active fractions from step a) onto a high performance weak cation exchange liquid chromatography column and eluting active fractions containing human immune interferon of enhanced purity from the column by use of a gradient of increasing salt concentration;

c)    passing pooled active fractions from step b) onto a silica gel permeation column and eluting active fractions containing human interferon of enhanced purity from the column by use of a buffered solution containing a low salt concentration and, if necessary,

d)    recycling pooled active fractions from step c) through step b) or step c) to provide homogeneous human interferon.

7.    The method of claim 6 wherein said affinity column in process step a) is concanavalin-A Sepharose, the crude human immune interferon is naturally occurring material and the active fractions are eluted with a buffered α-methyl-D-mannoside solution.

8.    The method of claim 6 wherein said weak cation exchange liquid chromatography column of step b) is carboxymethyl silica and said column is eluted with a 0-1M gradient of KCl in potassium phosphate buffer, pH 7.5.

9.    The method of claim 6 wherein said silica gel permeation column is eluted with a solution which is 0.3 M KCl in 25 mM potassium phosphate buffer, pH 7.5.

10. A process for the preparation of a parenteral dosage form which process comprises mixing homogeneous human immune interferon with a carrier suitable for parenteral application and therapeutically compatible adjuvants and bringing the resulting mixture into a suitable dosage form including, if desired, lyophilization of an aqueous solution.